# EUROPEAN PATENT SPECIFICATION

(11) **EP 0 595 927 B1**
(45) Date of publication and mention of the grant of the patent: **13.11.1996**
(21) Application number: 92915905.1
(22) Date of filing: 20.07.1992
(51) Int. Cl.: A61F 13/15, A61F 13/46

(54) **METHOD FOR CO-FORMING AN ABSORBENT STRUCTURE HAVING A TRANSFER LAYER AND A RESERVOIR LAYER**
VERFAHREN ZUM EINHEITLICHEN HERSTELLEN EINER ABSORBIERENDEN ANORDNUNG MIT EINER TRANSFER- UND EINER SPEICHERSCHICHT
PROCEDE DE FORMATION EN UNE SEULE ETAPE D'UNE STRUCTURE ABSORBANTE POSSEDANT UNE COUCHE DE TRANSFERT ET UNE COUCHE RESERVOIR

(30) Priority: 19.07.1991 US 732564
(43) Date of publication of application: 11.05.1994
(73) Proprietor: JOHNSON & JOHNSON INC., Montreal, Quebec H1N 2G4 (CA)
(72) Inventor: CHAUVETTE, Gaetan, St-Hubert, Quebec J3Y 8Z3 (CA); RAMACIERI, Patricia, Montreal, Quebec H1E 3J4 (CA); LEVESQUE, Yvon, Montreal, Quebec H1W 2E6 (CA)
(74) Representative: Strehl Schübel-Hopf Groening & Partner
(86) International application number: CA9200308
(87) International publication number: WO9301778

(56) References cited:
- EP-A- 0 108 637
- EP-A- 0 151 018
- EP-A- 0 359 501
- DE-A- 3 119 907
- US-A- 4 507 122
- US-A- 4 559 050
- US-A- 4 605 402

## Description

### FIELD OF THE INVENTION

The invention relates to the art of manufacturing structures for absorbing body exudate. More specifically, the invention relates to a simple and cost effective method for manufacturing a structurally integral, compound fluid-absorbent structure containing peat moss material, which is usually absorbent and retentive, and has a comparatively short fluid penetration time. The invention also extends to a fluid-absorbent article such as a sanitary napkin, a diaper, a urinary pad, an adult brief, a wound dressing and the like, incorporating the aforementioned fluid absorbent structure.

### BACKGROUND OF THE INVENTION

Many disposable absorbent articles use absorbent cores made primarily of cellulosic pulp fluff material. Such cores are generally soft, flexible and absorbent but tend to be bulky and thick and have poor wicking properties. In addition, cellulosic pulp fluff material has poor structural stability which may cause the absorbent core to collapse when saturated with fluid.

An absorbent structure that has poor wicking properties may increase the likelihood of failure of the absorbent product to hold and contain body fluids. Body exudate will be localized to a certain area of a poorly wicking absorbent core, causing saturation in such area whereby excess fluid may overflow through an external surface of the absorbent product. This overflow may contact the user's garment and cause stains or contact the user's body and cause wet discomfort or rash. It is therefore desirable to provide an absorbent core for disposable absorbent articles which can wick away body fluids from the point of contact with the absorbent core and spread it through the absorbent core to more efficiently utilize the entire surface area of the absorbent core. The improved wicking properties of such an absorbent core provide the capacity for fluids to travel by capillary pressure throughout the surface area of the absorbent core and thus permit the use of thinner cores, since more absorbent volume can be made available for absorbing body fluids by such wicking action. Thinner absorbent cores are more comfortable for the user and less unsightly or obvious when worn under clothes.

Absorbent cores with excellent wicking properties comprising peat moss and wood pulp composite materials are described, for example, in the following U.S. patents:

| PATENT # | INVENTOR(s) | DATE OF ISSUE |
|---|---|---|
| 4,170,515 | Lalancette et al. | October 9, 1979 |
| 4,215,692 | Levesque | August 5, 1980 |
| 4,226,237 | Levesque | October 7, 1980 |
| 4,305,393 | Nguyen | December 15, 1981 |
| 4,473,440 | Ovans | September 25, 1984 |
| 4,507,122 | Levesque | March 26, 1985 |
| 4,618,496 | Brasseur | October 21, 1986 |
| 4,676,871 | Cadieux et al. | June 30, 1987 |
| 4,992,324 | Dubé | February 12, 1991 |
| 5,053,029 | Yang | October 1, 1991 |

In accordance with the teaching of these patents, an absorbent structure comprising peat moss as a primary absorbent component is formed as a sheet by air or wet laying of fibers. The sheet is calendered to obtain a relatively thin, i.e. from about 0.025 to 0.25 centimeters (cm) thick and relatively dense, i.e. from about 0.2 to 1.0 grams per cubic centimeter (g/cc) structure. Such absorbent peat moss sheet may be processed to increase its flexibility for enhancing its comfort potential by subjecting the sheet to mechanical tenderizing such as by a perf-embossing process.

The peat moss sheet thus formed has a large proportion of extremely tiny pores and capillaries allowing the sheet to absorb and retain an enormous capacity of fluid. The peat moss pores swell as they absorb fluid, however, this swelling does not cause a loss of capacity for further absorbing fluid. Rather, the swelling contributes to the ability of the sheet to retain fluid while generally maintaining the structural integrity of the absorbent structure in use.

The wicking properties of the above-described peat moss sheet provide the ability for the sheet to be highly absorbent while remaining relatively thin.

Although peat moss material has certain highly desirable fluid absorption properties, it is characterized by a relatively slow fluid penetration time. This drawback is particularly significant for applications where the rate of fluid release is high. Urinary incontinence is an example where the onrush of body fluid can be contained only by an absorbent structure which exhibits an ultra-short fluid penetration time.

In order to adapt a peat moss absorbent layer for such applications, it is common practice to provide a highly permeable, fibrous, fluid transfer layer on the peat moss layer, whose function is to quickly collect and then meter the fluid to the peat moss layer. Fluid discharged on such composite absorbent structure will rapidly ingress the transfer layer due to its highly porous network. From the transfer layer, fluid migrates toward the peat moss layer by capillary pressure as a result of the substantial difference in wicking power therebetween. The fluid migration is well controlled, occurring at the rate of acceptance of the peat moss material.

US-A-4,559,050 discloses a method in accordance with the features of the preamble of claim 1. According to this known method, a nonwoven web of synthetic, wet resilient fibers containing at least about 200 percent by weight of superabsorbent is dried to a moisture content of less than about 25% and microcorrugated to provide a lower stiffness. The microcorrugating process consists of passing the web through fluted intermeshing rolls having sufficient pressure to fracture the superabsorbent and form cross-directional hinge lines. Subsequently, the web can be passed through an embossing roll with rings set to fracture the superabsorbent and place lines in the product in a machine direction, thus providing hinge lines in the machine direction. The fibrous web which contains the superabsorbent and forms a basic absorbent layer for the absorbent structure is of substantially high loft and upon dry compression followed by release has a tendency to return substantially to its original thickness. Fibrous webs formed from synthetic staple fibers are preferred. However, cellulosic fibers may also be used. The fibers are air-laid or melt-blown to form a web and is then stabilized. The stabilizing process is selected according to the fibers used and the process used to form the web. Suitable procedures for forming a web include carding, wet-laying, etc. According to one embodiment, a blend of staple polyester fibers with a minor portion of usable fibers, are air-laid to form a web. The web is subsequently lightly bonded by passing hot air through the fibers making the fusible fibers tacky so as to stick to each other and the staple fibers to provide some degree of integrity to the web structure.

Compound absorbent structures which include transfer and reservoir layers are costly to manufacture with traditional processes because the individual layers of the absorbent structure are manufactured separately, then assembled and bonded to form a structurally integral product.

For a satisfactory operation of the compound absorbent structure, the bond between the transfer and the reservoir layers must be strong and extensive to provide an intimate fluid-communicative relationship allowing fluid in the transfer layer to easily migrate toward the reservoir layer under the effect of capillary pressure.

Hence, the bonding of the transfer layer to the reservoir layer is a critical operation which must be performed with precision. As a result of the numerous steps required to assemble the compound absorbent structure and the accuracy required in executing these operations, the process is rendered complex and costly.

### OBJECTS OF THE INVENTION

An object of the invention is to provide a simple and a cost effective method for manufacturing a compound fluid-absorbent structure having a transfer layer and a reservoir layer containing peat moss material, which can accomplish the formation and bonding of the transfer and reservoir layers in a single operation.

### SUMMARY OF THE INVENTION

As embodied and broadly described herein, the invention provides a method for manufacturing a structurally integral, compound fluid-absorbent structure having superposed transfer and reservoir layers united in an intimate fluid-communicative relationship, the method comprising the steps of:
- laying in a superposed relationship and in physical contact an aqueous slurry of cellulosic fibrous material forming the transfer layer and an aqueous slurry of peat moss material forming the reservoir layer to form a laminated composite slurry layer;
- extracting dilution fluid from the laminated composite slurry layer to form a laminated superposed composite web including coextensive layers of cellulosic fibrous material and peat moss material which are intimately united to one another;
- mechanically compressing the laminated composite web to increase the density thereof;
- incorporating in the layer of cellulosic fibrous material an effective amount of debonding agent for reducing a cohesiveness of the cellulosic fibrous material;
- tenderizing by mechanical working the laminated composite web to donate to the laminated composite web enhanced flexibility, bulk and softness, the mechanical working causing a higher void volume (herein "void volume" shall mean the total volume of the interstices in a porous material) increase in the layer of cellulosic fibrous material due to the debonding agent therein than in the layer of peat moss material, whereby the mechanical working selectively affects the layer of cellulosic fibrous material for enhancing its ability to rapidly take-up fluid.

By using a co-forming process, the method for manufacturing the compound fluid-absorbent structure is greatly simplified because the formation and the bonding of the transfer and the reservoir layers is accomplished in a single operation. A further advantage of this method over conventional manufacturing techniques resides in the strength of the bond between the layers which is highly resistant to delamination. As a result, the compound fluid-absorbent structure can withstand intense mechanical working without loosing its structural integrity. Further, the bond is such as to establish the desirable intimate fluid-communicative relationship allowing fluid to easily migrate from the transfer layer toward the reservoir layer.

The method for manufacturing the compound fluid-absorbent structure in accordance with the invention comprises the step of mechanically compressing the laminated composite web, such as by calendering, for enhancing the drying power of the reservoir layer in order to enable the reservoir layer to efficiently desorb a fluid discharge captured by the transfer layer. The mechanical compression increases the density of the reservoir layer by reducing the amount of void volume therein. The primary objective is to reduce the average pore size and as a result to increase the capillary pressure exerted by the reservoir layer.

An undesirable effect of the calendering operation is to densify the transfer layer of the fluid-absorbent structure. This densification causes a reduction of its ability to rapidly take-up fluid and increases the risks of failure of the absorbent structure by overflow leakage.

To overcome this difficulty, the laminated composite web is subjected to mechanical working such as by perf-embossing and/or microcorrugating in order to increase the void volume of the transfer layer. The cellulosic material of the transfer layer is made responsive to this treatment by incorporating therein a debonding agent to inhibit the formation of hydrogen bonds between the cellulosic fibers, hence reducing the cohesiveness of the fibrous network. The debonding agent may be an agent acting chemically on the fibers or it may have a fibrous identity performing the function of a physical separator which neutralizes to some degree the hydrogen bonding by spacing apart the fibers of the cellulosic network.

The effect of the tenderizing operation on the reservoir layer is to increase its flexibility and softness, thereby enhancing its comfort potential. The perf-embossing operation also reduces to some degree the density of the peat moss network, however not to a point to significantly impair its ability to desorb the transfer layer.

In a most preferred embodiment, a slurry of cellulosic material containing the selected debonding agent is laid on a continuously advancing Fourdrinier wire. A slurry of peat moss material is then delivered on the slurry of cellulosic material to form the laminated composite slurry layer. The Fourdrinier wire is passed over a vacuum slot which establishes a pressure differential across the laminated composite slurry layer to extract water therefrom. The resulting web is dried, calendered and perf-embossed as previously discussed.

### BRIEF DESCRIPTION OF THE DRAWINGS

- Figure 1 is a perspective view of a structurally integral, composite, fluid-absorbent structure, constructed in accordance with the present invention;
- Figure 2 is a schematical representation of an apparatus for manufacturing the structurally integral, compound fluid-absorbent structure;
- Figure 3 is a graphic illustration of the perf-embossing operation for tenderizing the fluid-absorbent structure in accordance with the invention;
- Figure 4 is a vertical cross-sectional view of the perforation rolls which constitute the first stage of the perf-embossing treatment;
- Figure 5 is a fragmentary front elevational view of the perforation rolls shown in Figure 4, the fluid-absorbent structure to be treated being omitted for illustrating the interrelation between the perforation teeth on the rolls;
- Figure 6 is a vertical cross-sectional view of the cross-direction embossing rolls which constitute the second stage of the perf-embossing treatment;
- Figure 7 is a top view of one of the cross-direction embossing rolls, also showing the resulting embossing pattern created on the fluid-absorbent structure;
- Figure 8 is a vertical cross-sectional view of the machine direction embossing rolls which constitute third and last stage of the perf-embossing treatment;
- Figure 9 is a top view of one of the machine direction embossing rolls, also showing the resulting embossing pattern created on the fluid-absorbent structure;
- Figure 10 is a perspective view of a set-up for conducting a 45° impact capacity test procedure;
- Figure 11 is a perspective view of a set-up for conducting a penetration time test procedure; and
- Figure 12 is a fragmentary, perspective view of a sanitary napkin incorporating the fluid-absorbent structure in accordance with the invention as an absorbent core.

### DETAILED DESCRIPTION OF A PREFERRED EMBODIMENT

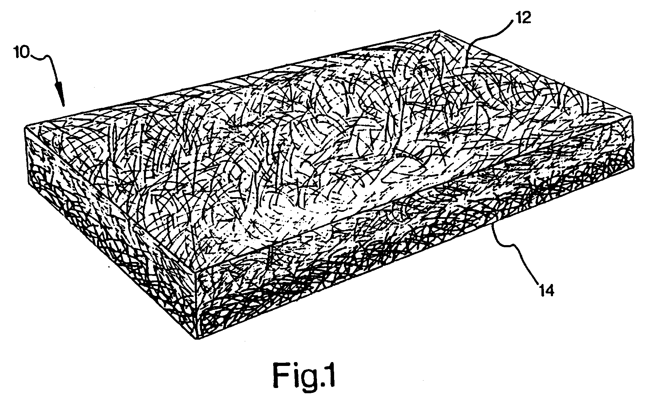

Referring to Figure 1, the reference numeral 10 designates comprehensively a compound, structurally integral fluid-absorbent structure embodying the principles of the present invention. Broadly stated, the absorbent structure 10 comprises a transfer layer 12 of hydrophilic cellulosic fibrous material intimately associated with a denser reservoir layer 14 comprising peat moss material. The absorbent structure 10 has a large capacity and a high fluid-absorption rate.

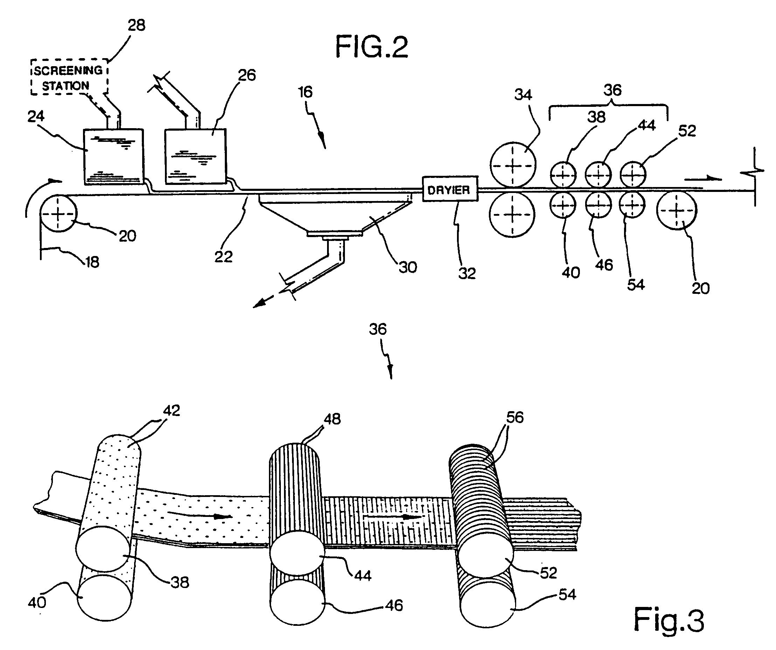

The detailed composition of the fluid-absorbent structure 10 will be best understood from the following description of the apparatus and the process for manufacturing such fluid-absorbent structure. Referring to Figure 2, the apparatus designated comprehensively by the reference numeral 16, comprises an endless, fluid pervious Fourdrinier wire 18 which is mounted on rollers 20 to provide a horizontally extending run 22 which is continuously advanced forward to support and convey a slurry of peat moss material and cellulosic fibers through various processing stations.

Headboxes 24 and 26 arranged in a spaced apart relationship along the path of travel of the wire 18, are provided to lay on the wire 18 slurry in sheeted form. The dual headbox arrangement deposits on the wire 18 two layers of slurry in a superposed relationship to form a laminated composite web. More specifically, the headbox 24 deposits on the wire 18 a slurry of peat moss material forming the reservoir layer 14 of the fluid-absorbent structure 10. The headbox 26 deposits on the slurry of peat moss material a slurry of cellulosic material forming the transfer layer 12.

The peat moss slurry, before being supplied to the headbox 24, is refined at a screening station illustrated schematically in Figure 2 and identified by the reference numeral 28. The screening station 28 is of a well-known construction and does not require a detailed description herein.

Downstream of the headboxes 24 and 26 is provided a vacuum slot 30 which is in fluid communication with a vacuum pump (not shown in the drawings) in order to create suction beneath the wire 18 for dewatering the slurry thereon.

The next process station is a dryer 32 whose purpose is to elevate the temperature of the web to evaporate water therefrom. The dryer is of a well-known construction and does not require a detailed description herein.

It may be envisaged to add between the dryer 32 and the vacuum slot a press section (not shown in the drawings) to express water from the web, as it is well known to those skilled in the art, in order to reduce the water contents of the web as much as possible before it is processed in the dryer 32.

Downstream of the dryer 32 is provided a calendering station 34 which mechanically compresses the dried product in order to densify the peat moss material for enhancing its drying power.

The calendering station is followed by a perf-embossing station 36 provided to tenderize the laminated composite web in order to relax the transfer layer which has been compacted at the calendering stage, and also to soften and render more flexible the laminated composite web for enhancing its comfort potential.

The mechanical tenderizing operation is graphically depicted in Figure 3. Generally stated, the perf-embossing technique first perforates the laminated composite web, then sequentially embosses the resulting material in the Y (cross-direction) and X (machine direction).

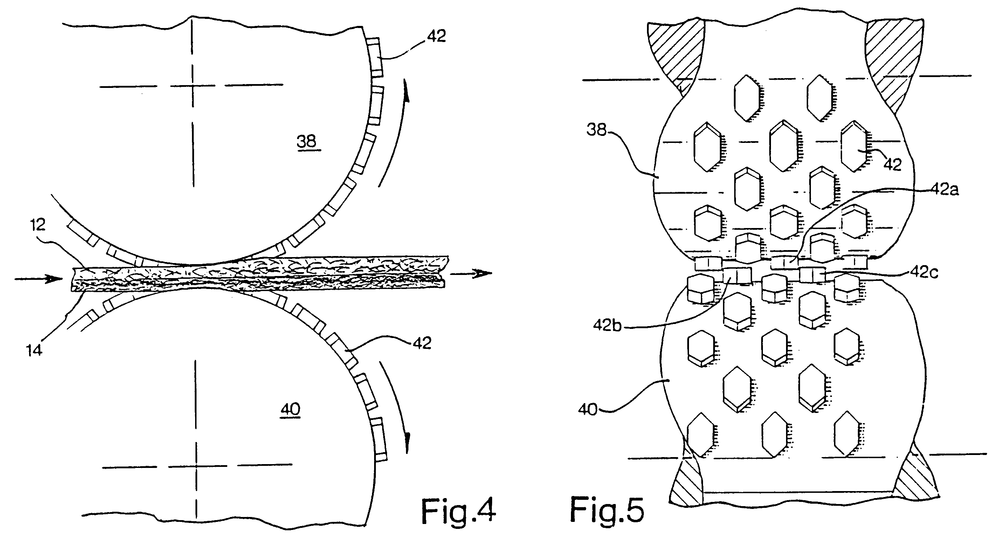

The "PERF" operation (first step), best illustrated in Figures 3, 4 and 5, is performed by passing the laminated composite web between a pair of rolls 38 and 40 provides with intermeshing and non-contacting teeth 42 perforating the material by shearing action to open-up its structure. The teeth 42 on the companion rolls 38,40 are so arranged that tooth 42a on the top roll 38 is off-center the inter-teeth void defined between adjacent and axially aligned teeth 42b and 42c. The shearing action actually occurs between teeth 42a,42c during intermeshing, locally perforating the laminated composite web.

In a preferred embodiment, the interference i.e. the overlap between the teeth 42 of the perforating rolls 38 and 40 is set at approximately 1.27 millimeters (mm). This setting may vary according to the thickness of the processed material and other factors.

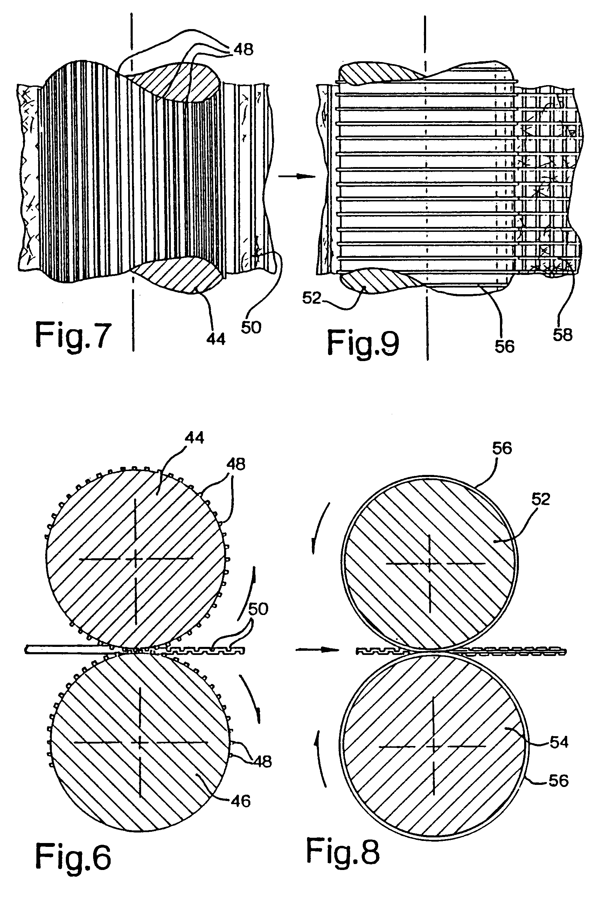

The second step of the perf-embossing operation consists of embossing the perforated, laminated composite web in the cross-direction by passing the web between a pair of rolls 44,46 with intermeshing longitudinally extending flutes 48. Figures 6 and 7 best illustrate the cross-direction embossing rolls 44 and 46 and the structure imparted to the laminated composite web. The flutes 48 imprint lines 50 on each surface of the material by locally compacting the material under the effect of mechanical compression.

In a preferred embodiment, the interference, i.e. overlap between the flutes 48 of the rolls 44,46 is set at approximately 0.89 mm. This setting may vary according to the specific operating conditions.

The last step of the perf-embossing operation consists in embossing the resulting material in the machine direction by passing the web between parallel rolls 52,54 having circumferentially extending and intermeshing flutes 56, as best shown in Figures 8 and 9. This means a perpendicular impact to the second step operation, creating longitudinal lines 58.

In a preferred embodiment, the interference between the machine direction embossing rolls 52,54 is set at 0.76 mm. This setting may vary according to the specific operating conditions.

In addition to relaxing the fibrous network of the transfer layer 12, the perf-embossing treatment contributes to provide other desirable mechanical properties to the laminated composite web, such as an increased flexibility enhancing the comfort potential of the product. The slits made at the perforation stage contribute to open-up the fibrous structure at precise locations, thus locally disrupting fiber bonds to render the material more pliable. The lines 50 and 58 constitute miniature hinges, extending throughout the entire surface of the non-defibrated cellulosic board to render the material more compliant in a transverse and in a longitudinal direction.

The operation of the apparatus 18 is as follows. The starting peat moss harvested from the bog should have a relatively high absorbent capacity. Peat moss capable of absorbing and retaining at least about 25 and preferably about 50 times its weight in water has been found satisfactory. The starting peat moss is wet classified at the screening station 28 to remove the extremely fine material, commonly referred to as fines, and large pieces of material including roots, branches and the like which do not contribute significantly to the absorbency of the peat moss material.

The classification is carried out such that anything that remains on a number 10 mesh screen (2000 microns) is discarded and anything that passes through a number 60 mesh screen (254 microns) is discarded.

The peat moss material is classified by a well-known wet screening process which consists of forming an aqueous slurry of the peat moss material and flowing the slurry through successive screens to extract from the slurry the fines and the excessively large particles.

The screened fraction of the peat moss material is then diluted with water to render the slurry more manageable. If desired, a fibrous component may be added at this stage to the slurry. The fibrous component may include such materials as Kraft wood pulp and mechanical wood pulp. As used herein, the term mechanical wood pulp is meant to include ground wood pulp, thermo-mechanical pulp and refiner wood pulp. Ground wood pulp is essentially threes and branches which have been debarked, cleaned and ground into particulate matter. Refiner wood pulp differs from ground wood pulp only in that the grinding step utilizes a refiner, i.e. a disk-like device well-known in the art and having metallic ribs at the peripheral sections thereof which last contact the wood particles and help separate the wood fibers without excessively damaging them. Thermo-mechanical wood pulp is similar to refiner pulp with the exception that the wood particles are heated in the refiner, usually with steam, to aid in separating the wood fibers. The common characteristic of these mechanical pulps is that no attempt has been made to separate the fibers by chemical means although they may later, after being reduced to fine particulate matter, be subjected to a desired chemical treatment, such as bleaching.

Preferably, when mechanical wood pulp is used in the peat moss slurry, such mechanical pulp has a Canadian Standard Freeness (TAPPI TEST METHOD T-227) of from about 60 to 750 and preferably from about 400 to 600.

The Kraft wood pulp, also usable in combination with the peat moss, is essentially chemically treated, long fibred wood pulp such as sulfite and sulfate wood pulps.

The fibrous component may also include a natural or synthetic textile fiber such as rayon, polyester, nylon, acrylic or the like, having a length of from about 0.6 cm to about 1.9 cm, preferably about 1.3 cm and a denier of from about 1.0 to 5.0, present in an amount from 2 to 20% by weight of the absorbent core 12, preferably from 4% to 8%.

The transfer layer 12 may be 100% cellulosic pulp or contain cellulosic pulp blended with polyester fibers, rayon fibers, cotton fibers or flexible foam (i.e. aminoether or low retention foam). Advantageously, the transfer layer 12 may be composed of a blend of cellulosic pulp with thermoplastic fibers for the purpose of stabilizing the transfer layer. For example, polyolefin fibers with the appropriate length and strength, such as low density polyethylene, or bicomponent fibers having polyethylene or polyester cores and a lower melting polyolefin sheath may be used, or polypropylene, polyvinylacetate, or other polyolefin fibers or thermoplastic pulp equivalents and the like. Blending such fibers with cellulosic pulp adds stability and integrity to the transfer layer material. The ratio of thermoplastic fiber to cellulosic pulp is preferably about 1:99 to about 50:50. More preferably, the ratio should be between about 3:97 and about 20:80. The fibers of the transfer layer may range in length from about 0.030 cm for ground wood pulp to about 7.5 cm for the stabilizing thermoplastic fibers. Preferably, the fibers are between about 0.60 cm to about 2.5 cm in length if the transfer layer 12 is intended to be stabilized by thermal bonding at the fibers' points of contact.

The slurry for making the transfer layer 12 is normally prepared by dispersing in water a cellulosic fibrous material such as sulfate, sulfite, debonded, bleached or unbleached wood pulp, thermo-mechanical wood pulp, chemical thermal mechanical pulp and mixtures thereof, to which is added the selected additive.

Preferably, the transfer layer 12 contains cross-linked cellulosic fibers in the range from about 20 to about 80 percent by weight of de-moisturized cellulose in the transfer layer 12. As discussed hereinafter, the cross-linked cellulosic fibers act as a debonding agent to reduce the cohesiveness of the fibrous network to render same more responsive to the perf-embossing operation.

The method for manufacturing the cross-linked cellulosic fibers 16 will not be described herein because this technology is well documented in the patent literature. For example, U.S. patent 4,853,086 granted to Weyerhaeuser Company on August 1, 1989, discloses a process for manufacturing the cross-linked cellulosic fibers by spraying a wet or a partially dried cellulosic fibrous web with an aqueous solution of a glycol and dialdehyde. The entire disclosure of this patent is incorporated herein by reference.

The transfer layer 12 may also contain a chemical debonding agent which can be used either in combination or in substitution to the cross-linked cellulosic fibers. Preferably, the amount of chemical debonding agent incorporated in the transfer layer 12 is in the range from about 0.05 to about 5 percent based on the weight of the de-moisturized transfer layer.

A chemical debonding agent which has been found satisfactory is commercialized under the BEROCELL 584 brand by the Berol Chemie Company.

Kraft wood pulp already treated with the BEROCELL 584 brand debonding agent in the range from about 0.3 to about 0.45 percent by weight of the demoisturized pulp, is a commercially available material which can be obtained for example from the Weyerhaeuser Company under the name NBFA. This product can be advantageously used as starting material for making the transfer layer slurry.

The slurry of peat moss material is first laid down on the wire 18 from the headbox 24. The slurry flow rate is selected to deliver on the wire 18 an amount of solids preferably in the range from about 50 grams per meter squared (g/m²) to about 700 g/m², more preferably from about 100 g/m² to about 500 g/m² and most preferably from about 175 g/m² to about 350 g/m². The consistency of the peat moss slurry is in the range from about 0.1 to about 0.5 percent by weight of de-moisturized solids in the slurry.

The peat moss slurry passes under the headbox 26 which delivers on the peat moss slurry a slurry of cellulosic material forming the transfer layer. The consistency of the slurry of cellulosic material is in the range from about 0.1 to about 0.5 percent by weight of de-moisturized solids in the slurry.

The flow rate of the slurry of cellulosic material is selected to deliver on the wire 18 an amount of solids in the range from 50 g/m² to about 400 g/m² and more preferably in the range from 75 g/m² to about 275 g/m².

The resulting laminated composite slurry layer is passed over the vacuum slot 30 to extract water under the influence of a pressure differential established across the slurry layer. It is necessary to regulate the residence time of the slurry layer over the vacuum slot and the vacuum intensity in order to control the density of the final product. Generally, decreased vacuum and increased speed will result in a less dense product. Conversely, increased vacuum and decreased speed will produce a denser product.

The laminated composite web, which leaves the dewatering station 30 passes through the dryer 32 and is then calendered at the calendering station 34 in order to densify the reservoir layer 14 to enhance its drying power. The calendering operation affects similarly the transfer layer which is not a desirable treatment as it reduces the void volume of the transfer layer and in turn adversely affects its ability to rapidly capture a fluid discharge.

To overcome this difficulty, the laminated compound web is treated at the perf-embossing station 36 which has the effect of softening the web in order to increase its comfort potential and most importantly to relax the fibrous network of the transfer layer, whereby, the latter can regain at least some of the void volume lost at the calendering station. To make the transfer layer more responsive to the perf-embossing treatment a debonding agent is incorporated therein, such as the cross-linked cellulosic fibers or a chemical debonding agent such as the BEROCELL 584 brand. If a debonding agent having a fibrous identity is desired, it is incorporated in the fibrous layer by adding the fibrous debonding agent to the slurry of starting material. A chemical debonding agent is more versatile because it can be incorporated at various stages of the manufacturing process. Most preferably, the starting material already treated with debonding agent is used such as Kraft wood pulp commercially available under the name NBFA, however it may also be envisaged to add the chemical debonding agent directly at the slurry either before or after the latter has been laid on the wire 18.

A hydrophilic chemical debonding agent is preferred. By "hydrophilic debonding agent" is meant a debonding agent which enhances the hydrophilicity of the cellulosic material forming the transfer layer. The BEROCELL 584 brand debonding agent is an example of such hydrophilic debonder.

An alternative to the perf-embossing technique is the microcorrugating operation which is similar to the perf-embossing except that no perforations are performed. The fluid-absorbent structure is solely subjected to an embossing operation to create closely spaced hinge lines. The microcorrugating operation is described in U.S. patent granted to Personal Product Company, numbers 4,596,567 and 4,559,050, issued on June 24, 1986 and December 17, 1988, respectively.

The invention will now be illustrated by examples. The examples are not intended to be limiting of the scope of the present invention but read in conjunction with a detailed and general description above provide a further understanding of the invention.

The fluid absorbency characteristics of the sample materials obtained by the following examples are reported in the section entitled "EXPERIMENTAL DATA". The various test procedures to which the sample materials have been subjected for the purpose of characterization are described in the section entitled "TEST PROCEDURES".

### EXAMPLE 1

A slurry of peat moss material having the following composition by weight of de-moisturized solids is prepared:

| | |
|---|---|
| sphagnum peat moss | = 74.3% |
| Kraft wood pulp fibers | = 21.0% |
| polyethylene fibers | = 4.7% |

A slurry of cellulosic material is prepared having the following composition by weight of demoisturized solids:

| | |
|---|---|
| - NBFA brand wood pulp (treated with debonding agent): | 25% |
| - Cross-linked cellulosic fibers (prepared in accordance with the teaching of the U.S. patent 4,853,086 mentioned earlier: | 75% |

A lay of 120 g/m² solids of the transfer layer slurry is deposited on a lay of 295 g/m² solids of the peat moss slurry. The resulting laminated composite slurry layer is dewatered, calendered and perf-embossed as discussed earlier.

### EXAMPLE 2

The same procedure set forth in example 1 is followed except that the formulation of the transfer layer slurry is modified as follows:

| | |
|---|---|
| - Cross-linked cellulosic fibers: | 50% |
| - Kraft wood pulp fibers treated with 1% with BEROCELL 584 brand and Debonding agent by weight of de-moisturized Kraft wood pulp fibers: | 50% |

by weight of de-moisturised solids in the slurry.

### EXAMPLE 3

The same procedure as set forth in example 1 is followed except that the formulation of the transfer layer slurry is changed as follows:

| | |
|---|---|
| - Cross-linked cellulosic fibers: | 75% |
| - Kraft wood pulp fibers treated with 0.5% BEROCELL 584 brand debonding agent by weight of de-moisturized Kraft wood pulp fibers: | 25% |

by weight of de-moisturized solids in the slurry.

### EXAMPLE 4

The same procedure as set forth in example 1 is followed except that the formulation of the transfer layer slurry is changed as follows:

| | |
|---|---|
| - Cross-linked cellulosic fibers treated with 1% BEROCELL 584 brand debonding agent based on the weight of de-moisturized cross-linked cellulosic fibers: | 75% |
| - Kraft wood pulp fibers treated with 1% BEROCELL 584 brand debonding agent based on the weight of de-moisturized Kraft wood pulp fibers: | 25% |

based on the weight of de-moisturized solids in the slurry.

### CONTROL

In order to provide a basis for comparison of the fluid absorption properties of the sample materials under examples 1 to 4, a control sample is provided which is constructed in accordance with the prior art. More particularly, a peat moss slurry having the composition set forth in claim 1 is prepared and a peat moss layer of this slurry is made having a basis weight of 295 g/m² (solids). On the top of the peat moss layer is mounted by adhesive or transfer layer which is an air-laid product manufactured and commercialized by the Merfin Company, having a basis weight of 120 g/m².

The materials of examples 1 to 4 and the control are tested to assess the following characteristics:
a) 45° impact capacity (expressed in percentage);
b) penetration time (expressed in seconds);
c) rewet/wet back (expressed in percentage).

### EXPERIMENTAL DATA

| Examples | 45° Impact Capacity (%) | Penetration time (sec) | Rewet/Wetback (%) |
|---|---|---|---|
| 1 | 94.4 | 41.4 | 249.0 |
| 2 | 76.4 | 65.8 | 143.6 |
| 3 | 80.8 | 62.6 | 212.2 |
| 4 | 86.2 | 43.6 | 151.0 |
| Control | 65.6 | 33.2 | 236.8 |

### TEST PROCEDURES

### 45° IMPACT CAPACITY

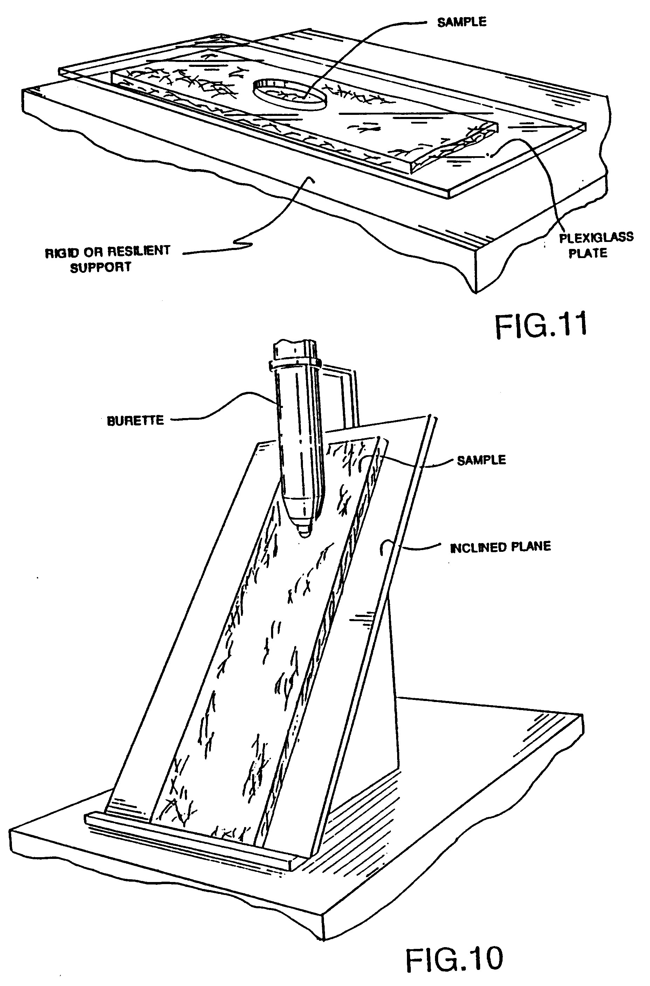

- Purpose:: To determine the fluid retention capacity of an absorbent material by measuring its ability to accept and retain a finite discharge of fluid at an inclined plane.
- Test procedure:: With reference to Figure 10, the impact capacity on a 5 cm by 25 cm sample is measured by weighing the amount of fluid that is retained in the sample placed on a 45° inclined plane, on which 25 cubic centimeters of test fluid has been released from an overhanging burette. The burette barely touches the sample at a point approximately 6.5 cm away from its upper extremity.
- Test fluid:: Synthetic menstrual fluid.

### PENETRATION TIME

- Purpose:: To determine the penetration time of an absorbent material by measuring the time required to completely absorb a finite amount of fluid.
- Test procedure:: The time required for a 5 cm X 20 cm sample under 0.276 kiloPascals (kPa) covered by a plexiglass plate, as shown in Figure 11, to absorb 15 cubic centimeters of test fluid fed to the sample through an oval orifice on the plate measuring 3.8 cm X 1.9 cm. The penetration time is recorded when all free liquid has disappeared from the surface of the sample exposed by the oval orifice.
- Test fluid:: Synthetic menstrual fluid.

### REWET/WETBACK:

- Purpose:: The purpose of this test is to assess the propensity of a fluid present in an absorbent material under pressure to wet back an adjacent surface in contact therewith.
- Test Procedure:: The sample material to which has been delivered 10 cubic centimeters of test fluid is allowed to rest 15 minutes and it is covered with a NuGauze brand pad. A pressure of 10.48 kPa is applied over the NuGauze pad. After 3 minutes, the amount of fluid (mass) captured by the pad is measured and reported in percentage on the basis of the dry weight of the pad.
- Test Fluid:: Synthetic menstrual fluid.

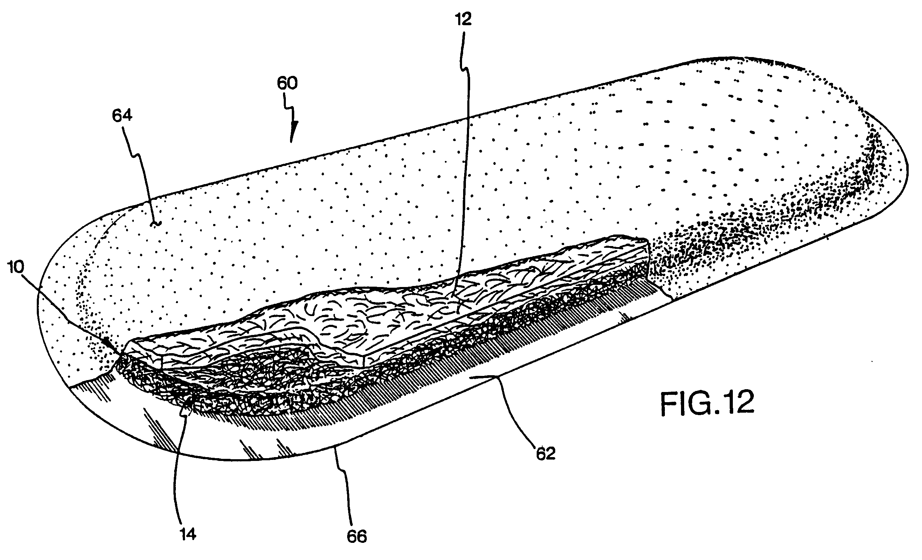

Figure 12 illustrates a sanitary napkin incorporating the absorbent structure according to the invention. The sanitary napkin, designated comprehensively by the reference numeral 60, comprises an envelope 62 defining an internal space receiving the absorbent structure 10. The envelope 62 includes a fluid-permeable cover layer 64 made of a non-woven fabric or any other suitable porous web, and a liquid-impervious backing layer 66, made of polyethylene film for example. The cover and backing layers 64 and 66 are heat-sealed to one another along their marginal portions.

To attach the sanitary napkin 60 to the wearer's underpants, the liquid impervious backing layer 66 may be provided with adhesive zones covered with a pealable backing (not shown in the drawings).

Sanitary napkins constructed in accordance with the present invention are found to possess a very high fluid absorption capacity and a comparatively high fluid penetration rate which reduces the risk of failure when a large quantity of fluid is suddenly released on the sanitary napkin.

Applications of the methods of the present invention for sanitary and other health-care uses can be accomplished by any sanitary protection, incontinence, medical and absorbent methods and techniques as are presently or prospectively known to those skilled in the art. Thus, it is intended that the present application covers the modifications and variations of this invention provided that they come within the scope of the appended claims and their equivalents.

## Claims

1. A method for manufacturing a structurally integral compound fluid-absorbent structure (10) having superposed transfer layer (12) of cellulosic fibers and reservoir layer (14) of peat moss material in an intimate fluid-communicative relationship characterized by the steps of:
- laying in a superposed relationship and in physical contact an aqueous slurry of material forming said transfer layer (12) and an aqueous slurry of material forming said reservoir layer (14) to form a laminated composite slurry layer;
- extracting dilution fluid from said laminated composite slurry layer to form a laminated composite web including superposed coextensive layers (12;14) of cellulosic fibrous material and peat moss material which are intimately united to one another;
- mechanically compressing said laminated composite web to increase the density thereof;
- incorporating in said layer of cellulosic fibrous material an effective amount of debonding agent for reducing a cohesiveness of said cellulosic fibrous material;
- tenderizing by mechanical working said laminated composite web to donate to said laminated composite web enhanced flexibility bulk and softness, said mechanical working causing a higher void volume increase in said layer (12) of cellulosic fibrous material due to said debonding agent therein than in said layer (14) of peat moss material, whereby said mechanical working selectively affects said layer (12) of cellulosic fibrous material in order to enhance its ability of rapidly taking-up fluid.

2. A method as defined in claim 1, wherein said mechanical working is a process selected from the group consisting of perf-embossing and microcorrugating.

3. A method as defined in claim 1, comprising the step of calendering said laminated composite web to mechanically compress same.

4. A method as defined in claim 1, comprising the step of incorporating said debonding agent in said cellulosic fibrous material before forming said slurry of cellulosic fibrous material.

5. A method as defined in claim 1, comprising the step of adding said debonding agent in said slurry of cellulosic fibrous material.

6. A method as defined in claim 1, wherein said debonding agent is hydrophilic.

7. A method as defined in claim 1, wherein said debonding agent has a fibrous identity.

8. A method as defined in claim 7, wherein said debonding agent comprises cellulosic fibers.

9. A method as defined in claim 8, wherein said debonding agent comprises cross-linked cellulosic fibers.

10. A method as defined in claim 9, comprising the step of incorporating said cross-linked cellulosic fibers in said layer (12) of cellulosic fibrous material in the range from about 20 to about 80 percent by weight of de-moisturized cellulose in said layer (12) of cellulosic fibrous material.

## Patentansprüche

1. Verfahren zur Herstellung einer strukturell integralen, fluidabsorbierenden Verbundstruktur (10), bei der übereinander eine Übertragungsschicht (12) aus Cellulosefasern und eine Reservoirschicht (14) aus Torfmaterial in einer engen Fluidverbindungsbeziehung angeordnet sind, gekennzeichnet durch folgende Stufen:
- eine wäßrige Aufschlämmung eines die Übertragungsschicht (12) bildenden Materials und eine wäßrige Aufschlämmung des die Reservoirschicht (14) bildenden Materials werden übereinander und in physikalischem Kontakt miteinander unter Bildung einer laminierten Verbundaufschlämmungsschicht angeordnet;
- Verdünnungsflüssigkeit wird aus der laminierten Verbundaufschlämmungsschicht extrahiert, wodurch eine laminierte Verbundbahn mit übereinander angeordneten, sich gemeinsam erstreckenden Schichten (12, 14) aus Cellulose-Fasermaterial und Torfmaterial, die innig miteinander verbunden sind, gebildet wird;
- die laminierte Verbundbahn wird zur Erhöhung ihrer Dichte mechanisch zusammengepreßt;
- der Schicht aus dem Cellulose-Fasermaterial wird eine wirksame Menge eines bindungslösenden Mittels einverleibt, um die kohäsive Beschaffenheit des Cellulose-Fasermaterials zu verringern;
- die Verbundbahn wird durch mechanische Bearbeitung mürbe gemacht, um Flexibilität, Volumen und Weichheit der Verbundbahn zu erhöhen, wobei die mechanische Bearbeitung in der Schicht (12) aus Cellulose-Fasermaterial aufgrund des bindungslösenden Mittels eine höhere Zunahme des Hohlraumvolumens bewirkt als in der Schicht (14) aus Torfmaterial, wobei die mechanische Bearbeitung selektiv die Schicht (12) aus Cellulose-Fasermaterial beeinflußt, um deren Fähigkeit zur raschen Flüssigkeitsaufnahme zu verstärken.

2. Verfahren nach Anspruch 1, wobei es sich bei der mechanischen Bearbeitung um einen Vorgang handelt, der aus der Gruppe perforierende Prägung und Mikroriffelung ausgewählt ist.

3. Verfahren nach Anspruch 1, umfassend die Stufe des Kalandrierens der laminierten Verbundbahn, um sie mechanisch zusammenzupressen.

4. Verfahren nach Anspruch 1, umfassend die Stufe des Einverleibens des bindungslösenden Mittels in das Cellulose-Fasermaterial vor der Bildung der Aufschlämmung des Cellulose-Fasermaterials.

5. Verfahren nach Anspruch 1, umfassend die Stufe der Zugabe des bindungslösenden Mittels zu der Aufschlämmung des Cellulose-Fasermaterials.

6. Verfahren nach Anspruch 1, wobei das bindungslösende Mittel hydrophil ist.

7. Verfahren nach Anspruch 1, wobei das bindungslösende Mittel eine faserige Beschaffenheit aufweist.

8. Verfahren nach Anspruch 7, wobei das bindungslösende Mittel Cellulosefasern umfaßt.

9. Verfahren nach Anspruch 8, wobei das bindungslösende Mittel vernetzte Cellulosefasern umfaßt.

10. Verfahren nach Anspruch 9, umfassend die Stufe des Einverleibens der vernetzten Cellulosefasern in die Schicht (12) aus Cellulose-Fasermaterial in einer Menge im Bereich von 20 bis 80 Gew.-% der entfeuchteten Cellulose in der Schicht (12) aus Cellulose-Fasermaterial.

## Revendications

1. Procédé de fabrication d'une structure d'absorption de fluide composée formée d'un seul tenant (10) ayant une couche de transfert (12) en fibres cellulosiques et une couche réservoir (14) en sphaigne superposées en une relation étroite de communication de fluide, caractérisé par les étapes consistant à :
déposer, en relation superposée et avec contact physique, une suspension aqueuse de matière formant ladite couche de transfert (12) et une suspension aqueuse de matière formant ladite couche réservoir (14) pour former une couche de suspension composite stratifiée ;
extraire le fluide de dilution de ladite couche de suspension composite stratifiée pour former un tissu composite stratifié comprenant des couches coextensives superposées (12 ; 14) de matière fibreuse cellulosique et de matière de sphaigne qui sont intimement unies l'une à l'autre ;
comprimer mécaniquement ledit tissu composite stratifié pour en augmenter la densité ;
incorporer dans ladite couche de matière fibreuse cellulosique une quantité efficace d'agent anti-adhésif pour réduire une cohésivité de ladite matière fibreuse cellulosique ;
attendrir ledit tissu composite stratifié en le travaillant mécaniquement pour conférer audit tissu composite stratifié une souplesse, un volume et une douceur améliorés, ledit travail mécanique entraînant une augmentation plus importante du volume de vide dans ladite couche (12) de matière fibreuse cellulosique du fait dudit agent anti-adhésif compris dedans que dans ladite couche (14) de matière de sphaigne, de telle sorte que ledit travail mécanique affecte de manière sélective ladite couche (12) de matière fibreuse cellulosique afin d'augmenter sa faculté d'absorption rapide de fluide.

2. Procédé selon la revendication 1, dans lequel ledit travail mécanique est un procédé choisi dans le groupe se composant du gaufrage par perforation et de la micro-ondulation.

3. Procédé selon la revendication 1, comprenant l'étape consistant à calandrer ledit tissu composite stratifié pour comprimer mécaniquement celui-ci.

4. Procédé selon la revendication 1, comprenant l'étape consistant à incorporer ledit agent anti-adhésif dans ladite matière fibreuse cellulosique avant de former ladite suspension de matière fibreuse cellulosique.

5. Procédé selon la revendication 1, comprenant l'étape consistant à ajouter ledit agent anti-adhésif à ladite suspension de matière fibreuse cellulosique.

6. Procédé selon la revendication 1, dans lequel ledit agent anti-adhésif est hydrophile.

7. Procédé selon la revendication 1, dans lequel ledit agent anti-adhésif a une identité fibreuse.

8. Procédé selon la revendication 7, dans lequel ledit agent anti-adhésif comprend des fibres cellulosiques.

9. Procédé selon la revendication 8, dans lequel ledit agent anti-adhésif comprend des fibres cellulosiques réticulées.

10. Procédé selon la revendication 9, comprenant l'étape consistant à incorporer lesdites fibres cellulosiques réticulées dans ladite couche (12) de matière fibreuse cellulosique dans la plage allant d'environ 20 à environ 80 pourcent en poids de cellulose déshumidifiée dans ladite couche (12) de matière fibreuse cellulosique.
